# EUROPÄISCHE PATENTSCHRIFT

(11) **EP 3 056 901 B1**
(45) Veröffentlichungstag und Bekanntmachung des Hinweises auf die Patenterteilung: **29.08.2018**
(21) Anmeldenummer: 15075007.3
(22) Anmeldetag: 13.02.2015
(51) Int. Cl.: G01N 33/53, G01N 33/68

(54) **Verfahren zum Nachweis von Proteinen mit Hilfe von Aptameren**
Method for the detection of proteins by means of aptamers
Procédé de contrôle de protéines à l'aide d'aptamères

(43) Veröffentlichungstag der Anmeldung: 17.08.2016
(73) Patentinhaber: Technische Universität Berlin, 10623 Berlin (DE)
(72) Erfinder: Kurreck, Jens, 12205 Berlin (DE); Wagner, Anke, Berlin (DE); Röhrs, Viola, 14197 Berlin (DE)
(74) Vertreter: Gulde & Partner

(56) Entgegenhaltungen:
- WO-A1-2011/060557
- WO-A2-00/73501
- US-A1- 2014 148 356
- SHIN SEONMI ET AL: "An alternative to Western blot analysis using RNA aptamer-functionalized quantum dots", BIOORGANIC & MEDICINAL CHEMISTRY LETTERS, Bd. 20, Nr. 11, 14. April 2010 (2010-04-14), Seiten 3322-3325, XP029120899, ISSN: 0960-894X, DOI: 10.1016/J.BMCL.2010.04.040
- "Best Practices: Western Blot", , 12. August 2014 (2014-08-12), Seiten 1-4, XP055203732, Gefunden im Internet: URL:http://www.basepairbio.com/wp-content/ uploads/2014/09/BP-Western-Blot-8.12.14-re v2.pdf [gefunden am 2015-07-21]
- KYUNG-MI SONG ET AL: "Aptamers and Their Biological Applications", SENSORS, Bd. 12, Nr. 12, 9. Januar 2012 (2012-01-09), Seiten 612-631, XP055152891, DOI: 10.3390/s120100612
- RHIE A ET AL: "Characterization of 2'-fluoro-RNA aptamers that bind preferentially to disease-associated conformations of prion protein and inhibit conversion", JOURNAL OF BIOLOGICAL CHEMISTRY, AMERICAN SOCIETY FOR BIOCHEMISTRY AND MOLECULAR BIOLOGY, US, Bd. 278, Nr. 41, 5. August 2003 (2003-08-05), Seiten 39697-39705, XP002401297, ISSN: 0021-9258, DOI: 10.1074/JBC.M305297200
- MORENO M ET AL: "Selection of aptamers against KMP-11 using colloidal gold during the SELEX process", BIOCHEMICAL AND BIOPHYSICAL RESEARCH COMMUNICATIONS, ACADEMIC PRESS INC. ORLANDO, FL, US, Bd. 308, Nr. 2, 22. August 2003 (2003-08-22), Seiten 214-218, XP004442963, ISSN: 0006-291X, DOI: 10.1016/S0006-291X(03)01352-4

### SEQUENCE LISTING

<110> Technische Universität Berlin
<120> Verfahren zum Nachweis von Proteinen mit Hilfe von Aptameren
<130> P12752EP-GL
<160> 2
<170> PatentIn version 3.5
<210> 1
   <211> 15
   <212> DNA
   <213> Artificial Sequence
<220>
   <223> Thrombin-bindendes Aptamer
<400> 1
   ggttggtgtg gttgg 15
<210> 2
   <211> 40
   <212> DNA
   <213> Artificial Sequence
<220>
   <223> Streptavidin-bindendes Aptamer
<400> 2
   atctccgatt gccccacgac gcagtggtcg gagttacttt 40

## Beschreibung

Die Erfindung betrifft ein Verfahren zum Nachweis von Proteinen mithilfe von Aptameren.

Im Stand der Technik sind diverse methodische Ansätze zum Nachweis von Proteinen bekannt. Breite Anwendung finden dabei beispielsweise Methoden wie ELISA (engl. Enzyme Linked Immunosorbent Assay), Dot Blot oder Western Blot, welche alle auf dem spezifischen Nachweis von Proteinen durch Antikörper basieren. Die Verwendung von Antikörpern bringt jedoch diverse Nachteile mit sich: Grundsätzlich ist die Produktion von Antikörpern ein komplizierter, zeitaufwändiger und kostenintensiver Prozess. Des Weiteren ist es für die Herstellung von polyklonalen bzw. monoklonalen Antikörpern notwendig entweder Tiere direkt für die Immunisierung zu verwenden oder aus ihnen Zellen für die Anwendung der Hybridom-Technologie zu gewinnen.

Es ist bekannt, dass Aptamere in vielen Anwendungen als Ersatz für Antikörper dienen können. Die Nutzung von Aptameren anstelle von Antikörpern bringt einige Vorteile mit sich, so erfolgt die Herstellung der Aptamere *in vitro,* d.h. dass im Gegensatz zur klassischen Antikörperherstellung auf die Nutzung von Tierversuchen oder tierischen Zellkulturen verzichtet werden kann. Da die Aptamere durch chemische Synthese produziert werden, gibt es außerdem keine Variationen zwischen den einzeln hergestellten Chargen. Durch den *in vitro* Selektionsprozess ist es außerdem möglich, Aptamere gegen Zielmoleküle zu produzieren, die zytotoxisch oder wenig immunogen sind.

Aptamere können wie Antikörper auch mit Marker- bzw. Reportermolekülen modifiziert werden, um sie in den verschiedensten methodischen Anwendungen detektieren zu können. So sind bereits Nachweisverfahren basierend auf biotinylierten Aptameren, radioaktiv- oder Fluoreszenz-markierten Aptameren oder Aptamer-fusionierten Nanopartikeln im Stand der Technik bekannt. Beispielsweise wird in WO 2000/73501 A2 die Verwendung von radioaktivmarkierten Aptameren zum Nachweis von Proteinen offenbart, die über ein natives PAA-Gradientengel aufgetrennt und anschließend geblottet wurden.

Eine große Herausforderung bei der Etablierung von Aptamer-Nachweismethoden, insbesondere bei Blotanwendungen, ist die Optimierung der Spezifität. So führt beispielsweise die Nutzung von Nanopartikeln in der Regel zu einem starken unspezifischen Hintergrund, der wahrscheinlich auf die Tatsache zurückzuführen ist, dass Nanopartikel ebenfalls mit Proteinen interagieren können. Auch der Nachweis von biotinylierten Aptameren mittels Streptavidin-HRP kann zu unspezifischen Signalen insbesondere in heterogenen Proteingemischen führen, da Streptavidin stark mit Biotin interagiert und somit auch biotinylierte Proteine detektiert werden.

Aufgabe der vorliegenden Erfindung ist es, einen oder mehrere Nachteile im Stand der Technik zu vermindern oder zu vermeiden. Insbesondere ist es eine Aufgabe der vorliegenden Erfindung ein Verfahren zur Verfügung zu stellen, mit dem ein spezifischer Nachweis eines Proteins in einer Probe möglich ist.

Ein Aspekt der vorliegenden Erfindung ist die Bereitstellung eines Verfahrens zum *in vitro* Nachweis eines ersten Proteins in einer Probe gemäß einem der Patentansprüche, umfassend die Schritte: a) Auftrennen der Probe mittels eines nativen Trennverfahrens; b) Transfer der aufgetrennten Probe auf eine Membran; c) in Kontakt bringen der Membran mit einem Aptamer, das spezifisch an das erste Protein bindet, wobei das in Kontakt bringen bei einer Temperatur von ≥ 30°C stattfindet; und d) Nachweis des ersten Proteins mittels Detektion des Aptamers, das an das erste Protein gebunden vorliegt.

Überraschenderweise wurde festgestellt, dass das in Kontakt bringen der Membran mit einem Aptamer bei einer Temperatur von ≥30°C einen spezifischeren Nachweis von Proteinen in einer Probe ermöglicht.

Soweit nachfolgend der Kontext nicht eindeutig etwas anderes ergibt, ist bei der Verwendung von Singular-Formen bzw. Plural-Formen stets sowohl die Ein- als auch die Mehrzahl umfasst.

Das erfindungsgemäße Verfahren bezieht sich auf ein Verfahren zum *in vitro* Nachweis eines Proteins in einer Probe. Mithilfe des erfindungsgemäßen Verfahrens ist es sowohl möglich eine qualitative Aussage über das Vorhandensein oder die Abwesenheit des Proteins in der Probe zu treffen als auch eine quantitative bzw. semiquantitative Bestimmung der Menge des Proteins innerhalb dieser Probe durchzuführen. Bei dem ersten Protein, das in einer Probe identifiziert werden soll, kann es sich um ein Protein jeglicher Art bzw. Ursprungs handeln. So kann es sich bei dem ersten Protein beispielsweise um ein natürliches Protein aus einer tierischen, pflanzlichen, bakteriellen oder einer ähnlichen Quelle handeln oder es kann synthetischen Ursprungs sein. Bei dem ersten Protein kann es sich um ein Polypeptid, Oligopeptid oder auch um ein Peptid handeln. Es kann an weitere Moleküle oder Strukturen gebunden vorliegen wie z.B. als Membranprotein oder aber auch ein lösliches Protein wie z.B. ein Hormon sein. Auch kann das erste Protein beispielsweise enzymatische oder toxische Eigenschaften haben. Das erste Protein kann Modifizierungen aufweisen wie Phosphorylierungen, Hydroxylierungen, Methylierungen, Glykosylierungen oder ähnliches. Vorzugsweise handelt es sich bei dem ersten Protein um ein humanes Protein, besonders bevorzugt um ein lösliches Protein, insbesondere bevorzugt um α-Thrombin.

Der Begriff "Probe" bezeichnet eine zu untersuchende Zusammensetzung, die für die *ex vivo* oder *in vitro* Untersuchung verwendet werden kann. Bei der Probe handelt es sich bevorzugt um biologisches oder medizinisches Material, also Material, das aus einem Organismus, von Bestandteilen eines Organismus oder aus Zellen gewonnen wird. Das Material kann, bevor es als Probe im erfindungsgemäßen Verfahren eingesetzt wird, weiteren Behandlungsschritten unterzogen werden, z.B. um das Material in einen Zustand zu versetzen, in dem es sich als Probe für das erfindungsgemäße Verfahren besonders eignet. Zur Aufbereitung können gängige Methoden verwendet werden, die dem Fachmann bekannt sind.

Vorzugsweise umfasst die Probe ein proteinhaltiges Gemisch. Das proteinhaltige Gemisch kann ein oder mehrere Proteine sowie weitere Bestandteile enthalten. Das proteinhaltige Gemisch kann aus einer Zellkultursuspension oder einem Zellkulturüberstand gewonnen werden oder eine Zellkultursuspension oder einen Zellkulturüberstand umfassen. Das proteinhaltige Gemisch kann außerdem aus einer zu untersuchenden Gewebeprobe erhalten werden oder eine Gewebeprobe umfassen. Bevorzugt handelt es sich dabei um eine Gewebeprobe aus einer Testperson, wobei die Testperson ein Tier sein kann, bevorzugt ein Säugetier, besonders bevorzugt ein Mensch. Des Weiteren kann das proteinhaltige Gemisch aus einer Körperflüssigkeit gewonnen werden oder eine Körperflüssigkeit umfassen. "Körperflüssigkeit" kann jede Flüssigkeit des Körpers darstellen z.B. Blut, Plasma, Serum, Synovialflüssigkeit, Urin, Stuhl, Interstitialflüssigkeit, Lymphe, Speichel, Schweiß, Spinalflüssigkeit, Tränenflüssigkeit etc.

Das Nachweisverfahren der vorliegenden Erfindung wird *in vitro* durchgeführt. Als *"in vitro"* wird jede Umgebung verstanden, die sich nicht innerhalb eines lebenden Organismus, z.B. einem menschlichen oder tierischen Körpers, befindet. Das erfindungsgemäße *in vitro* Nachweisverfahren umfasst also ausdrücklich kein Verfahren, welches am menschlichen oder tierischen Körper vorgenommen wird.

Beim erfindungsgemäßen Verfahren wird die Probe in einem ersten Verfahrensschritt a) mittels eines nativen Trennverfahrens aufgetrennt. Unter dem Begriff "Trennverfahren" wird im Rahmen der vorliegenden Erfindung eine Methode verstanden, welche die unterschiedlichen physikalischen und chemischen Eigenschaften von miteinander vermischten Komponenten ausnutzt, um diese voneinander zu trennen. Die Trennung der einzelnen Komponenten kann beispielsweise aufgrund ihrer unterschiedlichen Größe, Ladung, Löslichkeit oder isoelektrischen Punkte stattfinden. Dem Fachmann sind geeignete Methoden zur Trennung von Proben, welche zumindest ein erstes Protein enthalten, bekannt. Der Begriff "nativ" im Ausdruck "natives Trennverfahren" bedeutet, dass die natürliche Proteinfaltung des nachzuweisenden Proteins während dieses Trennverfahrens im Wesentlichen erhalten bleibt, d.h. es findet vornehmlich keine oder zumindest keine vollständige Denaturierung des Proteins statt so dass dessen native Sekundär-, Tertiär-, oder Quartärstruktur im Wesentlichen unbeeinflusst bleibt. Vorzugsweise handelt es sich bei dem nativen Trennverfahren um eine elektrophoretische Auftrennung, bevorzugt um eine Gelelektrophorese wie z.B. die native Polyacrylamid-Gelelektrophorese (PAGE) oder die isoelektrische Fokussierung (IEF). Insbesondere ist das native Trennverfahren dadurch charakterisiert, dass eine SDS-Konzentration von 0% bis 0,2 % SDS, bevorzugt 0,01% bis 0,15% SDS, besonders bevorzugt 0,1% SDS verwendet wird.

In einem nächsten Schritt des erfindungsgemäßen Verfahrens erfolgt der Transfer der aufgetrennten Probe auf eine Membran (Schritt b). Optional kann nach dem Transfer auf die Membran ein Blockieren der Membran erfolgen, damit freie unspezifische Bindungsstellen auf der Membran für eine Bindung unzugänglich gemacht werden. Die Blockierung der Membran kann beispielsweise mit Milchpulver, Albumin wie z.B. Rinderserumalbumin (BSA, bovine serum albumin), Gelatine oder ähnlichem durchgeführt werden. Geeignete Bedingungen unter denen der Transfer bzw. die Blockierung der Membran durchgeführt werden können sind dem Fachmann bekannt.

Im nachfolgenden Schritt c) des erfindungsgemäßen Verfahrens wird die Membran mit einem Aptamer in Kontakt gebracht, welches spezifisch an das erste Protein bindet.

Unter "Aptamer" wird im Sinne des erfindungsgemäßen Verfahrens ein einzelsträngiges Oligonukleotid eines Nukleinsäuremoleküls, wie beispielsweise einer Ribonukleinsäure (RNA, Abkürzung für englisch ribonucleic acid) oder einer Desoxyribonukleinsäure (ssDNA, Abkürzung für englisch single stranded desoxyribonucleic acid) verstanden. Umfasst das Aptamer eine Ribonukleinsäure, kann es als RNA-Aptamer bezeichnet werden. Wenn es hingegen eine Desoxyribonukleinsäure umfasst, kann es als DNA-Aptamer bezeichnet werden.

Aufgrund seiner speziellen dreidimensionalen Struktur bindet ein Aptamer spezifisch und mit hoher Affinität an sein Zielmolekül, z.B. an ein Protein in einer Probe. Der Begriff "spezifisch" oder "spezifische Bindung" bedeutet, dass das Aptamer unter den gewählten Bedingungen vorzugsweise selektiv an das nachzuweisende Zielmolekül binden kann.

Aptamere enthalten oder bestehen aus einer Sequenz von Nukleinsäuremolekülen, welche aus einzelnen Nukleotiden aufgebaut sind. Nukleotide bestehen aus drei Bestandteilen, nämlich aus Phosphaten, einem Zucker und einer Nukleobase. Der Begriff "Nukleotide" bezieht sich hierbei sowohl auf Ribonukleotide als auch auf Desoxyribonukleotide. Gemäß dem allgemeinen Ein-Buchstaben-Codes für Nukleobasen steht "A" für Adenin, "C" für Cytosin, "G" für Guanin, "T" für Thymin und "U" für Uracil, wobei in RNA-Aptameren die Nukleobase Thymin gegen Uracil ausgetauscht ist.

Bevorzugt ist das Aptamer spezifisch für ein erstes Protein.

Das in Kontakt bringen der Membran mit dem Aptamer beinhaltet vorzugsweise ein flüssiges Milieu und wird über einen Zeitraum durchgeführt, der die Ausbildung einer spezifischen Bindung zwischen dem ersten Protein und dem entsprechenden Aptamer erlaubt. Bevorzugt beträgt dieser Zeitraum länger als 30 Sekunden, besonders bevorzugt länger als 5 Minuten, ganz besonders bevorzugt beträgt der Zeitraum 5 Minuten bis 48 Stunden. Insbesondere bevorzugt ist eine Inkubationsdauer von 1 Stunde.

Unabhängig von der Art des Trennverfahrens in Schritt a) erfolgt das in Kontakt bringen der Membran mit dem Aptamer in Schritt c) des erfindungsgemäßen Verfahrens bei einer Temperatur von ≥30°C. Überraschenderweise wurde gefunden, dass sich die Inkubation der Membran bei einer, im Vergleich zur Raumtemperatur, erhöhten Temperatur vorteilhaft auf die Spezifität des Nachweises des Proteins auswirkt. So zeigen sich bei einer erhöhten Inkubationstemperatur ein deutlich reduziertes Hintergrundsignal der Membran an sich und ebenfalls geringere Signale von unspezifischen Bindungen. Vorzugsweise findet das in Kontakt bringen bei einer Temperatur von 30°C bis 70°C statt, bevorzugt bei 40°C bis 60°C, besonders bevorzugt bei 40°C bis 50°C. Insbesondere bevorzugt findet das in Kontakt bringen der Membran bei einer Temperatur von 50°C statt.

Im nachfolgenden Schritt d) des erfindungsgemäßen Verfahrens erfolgt der Nachweis des ersten Proteins mittels Detektion des Aptamers, welches an das erste Protein gebunden vorliegt. Die Detektion kann in einem oder in mehreren Schritten erfolgen. Es besteht die Möglichkeit, dass nach dem in Kontakt bringen der Membran mit dem Aptamer und vor dessen Detektion die Bestandteile aus dem Versuchsansatz entfernt werden, die nicht an das erste Protein gebunden haben, was z.B. durch einen oder mehrere Wasch-, Reinigungs- oder Isolierungsschritte erfolgen kann.

Diese Detektion kann direkt oder indirekt erfolgen. Eine direkte Detektion des Aptamers kann dann durchgeführt werden, wenn das Aptamer eine Modifikation mit einer Markierung aufweist, welche zum unmittelbaren Nachweis des markierten Aptamers verwendet werden kann. Dem Fachmann sind sowohl geeignete Markierungen als auch Methoden bekannt mit denen Aptamere modifiziert werden können. Bevorzugte Modifikationen sind z.B. Fluoreszenzmarkierungen, Radioisotop-Markierungen, Peroxidase-Markierungen, alkalische Phosphatase-Markierungen oder Markierungen mit Nanopartikeln. Bevorzugt weist das Aptamer eine Fluoreszenzmarkierung auf, besonders bevorzugt eine Markierung mit einem Fluorescein, insbesondere mit 6-Carboxyfluorescein. Ebenfalls bevorzugt ist eine Markierung mit einem Cyanin-Farbstoff, insbesondere mit Cy5.

Methoden zur direkten Detektion der modifizierten Aptamere sind dem Fachmann bekannt. Handelt es sich bei der Modifikation des Aptamers beispielsweise um eine Fluoreszenzmarkierung, kann durch Anregung des Fluorophors mit geeigneter Wellenlänge, die resultierende Fluoreszenz detektiert werden.

Vorzugsweise erfolgt die Detektion mithilfe einer enzymatischen, autoradiografischen oder Fluoreszenz-Nachweismethode, besonders bevorzugt mit einer Fluoreszenz-Nachweismethode.

Die Detektion des Aptamers kann auch indirekt erfolgen. Die indirekte Detektion kann beispielsweise durchgeführt werden, wenn das Aptamer eine Modifikation mit einer Markierung aufweist, welche zum mittelbaren Nachweis des markierten Aptamers verwendet werden kann. Bevorzugte Markierungen sind z.B. Biotin-Markierungen oder Digoxigenin-Markierungen. Vorzugsweise erfolgt die indirekte Detektion des Aptamers mithilfe eines Aptamer-spezifischen Antikörpers, der an das Aptamer gebunden vorliegt.

Der Aptamer-spezifische Antikörper kann eine Modifikation mit einer Markierung aufweisen, welche zum weiteren Nachweis des Aptamer-spezifischen Antikörpers verwendet werden kann. Dem Fachmann sind sowohl geeignete Markierungen als auch Methoden bekannt mit denen Aptamer-spezifische Antikörper modifiziert werden können. Bevorzugte Modifikationen sind z.B. Biotin-Markierungen, Fluoreszenzmarkierungen, Radioisotop-Markierungen, Digoxigenin-Markierungen, Peroxidase-Markierungen, alkalische Phosphatase-Markierungen oder Markierungen mit Nanopartikeln.

Mithilfe des erfindungsgemäßen Verfahrens kann ein erstes Protein in einer Probe nachgewiesen werden. Die vorliegende Erfindung umfasst auch ein Verfahren zum zusätzlichen *in vitro* Nachweis eines zweiten Proteins in der Probe, wobei zusätzlich zu den bereits beschriebenen Verfahrensschritten a) bis d) zum Nachweis eines ersten Proteins die weiteren Schritte i) und ii) durchgeführt werden, um ein zweites Protein in der Probe nachzuweisen. Bei der hierfür verwendeten Probe handelt es sich um dieselbe Probe, in der auch das erste Protein mithilfe des erfindungsgemäßen Verfahrens nachgewiesen wird. Entsprechend werden also in einer einzigen Probe ein erstes und ein zweites Protein nachgewiesen.

Zum Nachweis des zweiten Proteins in der Probe, wird nach Schritt b) des Verfahrens für den Nachweis des ersten Proteins, die Membran mit einem Bindungsmolekül, das spezifisch an das zweite Protein bindet, in Kontakt gebracht (Schritt i). Anschließend erfolgt der Nachweis des zweiten Proteins mittels Detektion des Bindungsmoleküls, das an das zweite Protein gebunden vorliegt (Schritt ii).

Schritt i) des Verfahrens zum Nachweis eines zweiten Proteins in der Probe kann dabei vor, während oder nach Schritt c) des Verfahrens zum Nachweis des ersten Proteins in der Probe erfolgen. In jedem Fall erfolgt Schritt i) nach Schritt b). Unabhängig davon kann Schritt ii) zum Nachweis des zweiten Proteins in der Probe dabei vor, während oder nach Schritt d) des Verfahrens zum Nachweis des ersten Proteins in der Probe erfolgen. Die genaue zeitliche Durchführung von Schritt i) hängt u.a. von der Art des ausgewählten Bindungsmoleküls bzw. von gewählten Versuchsbedingungen des Verfahrens ab.

Unter dem Begriff "zweites Protein" ist ein Protein zu verstehen, welches in den einzelnen Ausführungsformen nicht das gleiche Protein wie das erste Protein ist. Das bedeutet, dass, wenn das erste Protein z.B. ein α-Thrombin ist, dann kann das zweite Protein jedes andere Protein sein außer α-Thrombin. Beim zweiten Protein kann es sich auch um ein zweites Epitop des ersten Proteins handeln. Vorzugsweise handelt es sich bei dem zweiten Protein um Streptavidin.

Als "Bindungsmolekül" oder "Bindungsmoleküle" werden im erfindungsgemäßen Verfahren solche Substanzen bezeichnet, die unter den gewählten Bedingungen vorzugweise selektiv an das nachzuweisende zweite Protein binden können. Dem Fachmann sind geeignete Bindungsmoleküle als auch Verfahren zu deren Herstellung bekannt. Die Bindungsmoleküle können auf Polypeptiden, Poly-oder Oligonukleinsäuren oder ähnlichem basieren.

Bei dem Bindungsmolekül handelt es sich vorzugsweise um ein weiteres Aptamer. Die Definition des Ausdrucks "weiteres Aptamer" entspricht der Definition für "Aptamer" mit dem Vorbehalt, dass das weitere Aptamer in den einzelnen Ausführungsformen nicht die gleiche Spezifität aufweist wie das Aptamer. Das bedeutet also, dass, wenn das Aptamer z.B. eine Spezifität für ein erstes Protein aufweist, dann kann das weitere Aptamer jede andere Spezifität aufweisen außer jener für das erste Protein. Das weitere Aptamer und das Aptamer können sich auch aufgrund ihrer Epitop-Spezifität, Sequenz und/oder Markierung etc. voneinander unterscheiden.

Bevorzugt ist das weitere Aptamer spezifisch für ein zweites Protein.

Ebenfalls bevorzugt kann es sich bei dem Bindungsmolekül um ein Polypeptid handeln wie z.B. ein Zelladhäsionsmolekül aus der Gruppe der Integrine, Cadherine, Selektine oder um Mitglieder der Immunglobulin (Ig) Superfamilie. Bevorzugt handelt es sich bei dem Polypeptid um einen Antikörper, wobei sowohl ein polyklonaler und monoklonaler Antikörper mit klassischer Antikörperstruktur als auch davon abgeleitete Derivate oder Fragmente umfasst sein können. Solche Fragmente sind bekannt unter der Bezeichnung Fab, F(ab)₂, dsFv-Fragmente, scFV-Fragmente und single chain Antikörper. Vorzugsweise handelt es sich bei dem Bindungsmolekül um einen Antikörper.

In Schritt ii) des erfindungsgemäßen Verfahrens erfolgt der Nachweis des zweiten Proteins mittels Detektion des Bindungsmoleküls, welches an das zweite Protein gebunden vorliegt. Die Detektion kann in einem oder in mehreren Schritten erfolgen. Es besteht die Möglichkeit, dass nach dem in Kontakt bringen der Membran mit dem Bindungsmolekül (Schritt i)) und vor dessen Detektion in Schritt ii) die Bestandteile aus dem Versuchsansatz entfernt werden, die nicht an das zweite Protein gebunden haben, was z.B. durch einen oder mehrere Wasch-, Reinigungs- oder Isolierungsschritte erfolgen kann. Diese Detektion kann direkt oder indirekt erfolgen.

Eine direkte Detektion des Bindungsmoleküls kann dann durchgeführt werden, wenn das Bindungsmolekül eine Modifikation mit einer Markierung aufweist, welche zum weiteren Nachweis des markierten Bindungsmoleküls verwendet werden kann. Dem Fachmann sind sowohl geeignete Markierungen als auch Methoden bekannt mit denen Bindungsmoleküle modifiziert werden können. Bevorzugte Modifikationen sind z.B. Biotin-Markierungen, Fluoreszenzmarkierungen, Radioisotop-Markierungen, Digoxigenin-Markierungen, Peroxidase-Markierungen, alkalische Phosphatase-Markierungen oder Markierungen mit Nanopartikeln. Bevorzugt weist das Bindungsmolekül eine Fluoreszenzmarkierung auf, besonders bevorzugt eine Markierung mit einem Fluorescein, insbesondere mit 6-Carboxyfluorescein. Ebenfalls bevorzugt ist eine Markierung mit einem Cyanin-Farbstoff, insbesondere mit Cy5.

Methoden zur direkten Detektion der modifizierten Bindungsmoleküle sind dem Fachmann bekannt. Handelt es sich bei der Modifikation des Bindungsmoleküls beispielsweise um eine Fluoreszenzmarkierung, kann durch Anregung des Fluorophors mit geeigneter Wellenlänge, die resultierende Fluoreszenz detektiert werden. Vorzugsweise erfolgt die direkte Detektion mithilfe einer enzymatischen, autoradiografischen oder Fluoreszenz-Nachweismethode, besonders bevorzugt mit einer Fluoreszenz-Nachweismethode.

Die Detektion des Bindungsmoleküls kann auch indirekt erfolgen. Vorzugsweise erfolgt die indirekte Detektion des Bindungsmoleküls mithilfe eines Bindungsmolekül-spezifischen Antikörpers, der an das Bindungsmolekül gebunden vorliegt.

Der Bindungsmolekül-spezifische Antikörper kann eine Modifikation mit einer Markierung aufweisen, welche zum weiteren Nachweis des Bindungsmolekül-spezifischen Antikörpers verwendet werden kann. Dem Fachmann sind sowohl geeignete Markierungen als auch Methoden bekannt, mit denen Bindungsmolekül-spezifische Antikörper modifiziert werden können. Bevorzugte Modifikationen sind z.B. Biotin-Markierungen, Fluoreszenzmarkierungen, Radioisotop-Markierungen, Digoxigenin-Markierungen, Peroxidase-Markierungen, alkalische Phosphatase-Markierungen oder Markierungen mit Nanopartikeln.

In einer bevorzugten Ausführungsform umfasst das Verfahren zum *in vitro* Nachweis eines ersten Proteins in einer Probe die Schritte: a) Auftrennen der Probe mittels eines nativen Trennverfahrens; b) Transfer der aufgetrennten Probe auf eine Membran; c) in Kontakt bringen der Membran mit einem Aptamer, das spezifisch an das erste Protein bindet, wobei das in Kontakt bringen bei einer Temperatur von 40°C bis 60°C stattfindet; und d) Nachweis des ersten Proteins mittels Detektion des Aptamers, das an das erste Protein gebunden vorliegt, wobei das Aptamer eine Fluoreszenzmarkierung aufweist und die Detektion des markierten Aptamers direkt mithilfe einer Fluoreszenz-Nachweismethode erfolgt.

In einer weiteren bevorzugten Ausführungsform werden in einer Probe ein erstes Protein mithilfe eines Aptamers und ein zweites Protein mithilfe eines weiteren Aptamers *in vitro* nachgewiesen. Das erfindungsgemäße Verfahren umfasst dabei die Schritte: a) Auftrennen der Probe mittels eines nativen Trennverfahrens; b) Transfer der aufgetrennten Probe auf eine Membran; c) in Kontakt bringen der Membran mit einem Aptamer, das spezifisch an das erste Protein bindet, wobei das in Kontakt bringen bei einer Temperatur von 40°C bis 60°C stattfindet; sowie in Kontakt bringen der Membran mit einem weiteren Aptamer, das spezifisch an das zweite Protein bindet (Schritt i)); und d) Nachweis des ersten Proteins mittels Detektion des Aptamers, das an das erste Protein gebunden vorliegt sowie Nachweis des zweiten Proteins mittels Detektion des weiteren Aptamers, das an das zweite Protein gebunden vorliegt (Schritt ii)). Schritt i) kann dabei vor, während oder nach Schritt c) des Verfahrens durchgeführt werden. Des Weiteren kann Schritt ii) vor, während oder nach Schritt d) des Verfahrens stattfinden. Sowohl das Aptamer als auch das weitere Aptamer weisen eine Fluoreszenzmarkierung auf. Das markierte Aptamer und das markierte weitere Aptamer werden mittels einer direkten Detektion nachgewiesen, wobei die direkte Detektion eine Fluoreszenz-Nachweismethode ist.

In einer weiteren bevorzugten Ausführungsform werden in einer Probe ein erstes Protein mithilfe eines Aptamers und ein zweites Protein mithilfe eines Antikörpers als Bindungsmolekül *in vitro* nachgewiesen. Das erfindungsgemäße Verfahren umfasst dabei die Schritte: a) Auftrennen der Probe mittels eines nativen Trennverfahrens; b) Transfer der aufgetrennten Probe auf eine Membran; c) in Kontakt bringen der Membran mit einem Aptamer, das spezifisch an das erste Protein bindet, wobei das in Kontakt bringen bei einer Temperatur von 40°C bis 60°C stattfindet; sowie in Kontakt bringen der Membran mit einem Antikörper, der spezifisch an das zweite Protein bindet (Schritt i)); und d) Nachweis des ersten Proteins mittels Detektion des Aptamers, das an das erste Protein gebunden vorliegt sowie Nachweis des zweiten Proteins mittels Detektion des Antikörpers, der an das zweite Protein gebunden vorliegt (Schritt ii)). Schritt i) kann dabei vor, während oder nach Schritt c) des Verfahrens durchgeführt werden. Des Weiteren kann Schritt ii) vor, während oder nach Schritt d) des Verfahrens stattfinden.

In einer besonders bevorzugten Ausführungsform werden in einer Probe ein erstes Protein mithilfe eines Aptamers und ein zweites Protein mithilfe eines Antikörpers als Bindungsmolekül *in vitro* nachgewiesen. Das erfindungsgemäße Verfahren umfasst dabei die Schritte: a) Auftrennen der Probe mittels eines nativen Trennverfahrens; b) Transfer der aufgetrennten Probe auf eine Membran; c) in Kontakt bringen der Membran mit einem Aptamer, das spezifisch an das erste Protein bindet, wobei das in Kontakt bringen bei einer Temperatur von 40°C bis 60°C stattfindet; sowie in Kontakt bringen der Membran mit einem Antikörper, der spezifisch an das zweite Protein bindet (Schritt i)); und d) Nachweis des ersten Proteins mittels Detektion des Aptamers, das an das erste Protein gebunden vorliegt sowie Nachweis des zweiten Proteins mittels Detektion des Antikörpers, der an das zweite Protein gebunden vorliegt (Schritt ii)). Schritt i) kann dabei vor, während oder nach Schritt c) des Verfahrens durchgeführt werden. Des Weiteren kann Schritt ii) vor, während oder nach Schritt d) des Verfahrens stattfinden. Dabei weisen sowohl das Aptamer als auch der Antikörper eine Fluoreszenzmarkierung auf. Das markierte Aptamer und der markierte Antikörper werden mittels einer direkten Detektion nachgewiesen, wobei die direkte Detektion eine Fluoreszenz-Nachweismethode ist.

In einer weiteren besonders bevorzugten Ausführungsform werden in einer Probe ein erstes Protein mithilfe eines Aptamers und ein zweites Protein mithilfe eines Antikörpers als Bindungsmolekül in vitro nachgewiesen. Das erfindungsgemäße Verfahren umfasst dabei die Schritte: a) Auftrennen der Probe mittels eines nativen Trennverfahrens; b) Transfer der aufgetrennten Probe auf eine Membran; c) in Kontakt bringen der Membran mit einem Aptamer, das spezifisch an das erste Protein bindet, wobei das in Kontakt bringen bei einer Temperatur von 40°C bis 60°C stattfindet; sowie in Kontakt bringen der Membran mit einem Antikörper, der spezifisch an das zweite Protein bindet (Schritt i)); und d) Nachweis des ersten Proteins mittels Detektion des Aptamers, das an das erste Protein gebunden vorliegt sowie Nachweis des zweiten Proteins mittels Detektion des Antikörpers, der an das zweite Protein gebunden vorliegt (Schritt ii)). Schritt i) kann dabei vor, während oder nach Schritt c) des Verfahrens durchgeführt werden. Des Weiteren kann Schritt ii) vor, während oder nach Schritt d) des Verfahrens stattfinden. Dabei weist das Aptamer eine Fluoreszenzmarkierung auf und wird mittels direkter Detektion, vorzugsweise mithilfe einer Fluoreszenz-Nachweismethode, nachgewiesen. Die Detektion des Antikörpers erfolgt indirekt mithilfe eines weiteren Antikörpers, welcher spezifisch an den Antikörper bindet (sog. Bindungsmolekül-spezifischer Antikörper).

Es ist auch möglich mithilfe des erfindungsgemäßen Verfahrens neben dem Nachweis eines ersten und eines zweiten Proteins noch weitere Proteine in derselben Probe nachzuweisen.

Die vorliegende Offenbarung bezieht sich auch auf die Verwendung des erfindungsgemäßen Verfahrens zum *in vitro* Nachweis eines Proteins in einer Probe.

Außerdem bezieht sich die vorliegende Offenbarung auf die Verwendung des erfindungsgemäßen Verfahrens zur *in vitro* Diagnose einer Krankheit, welche über den Nachweis des Vorhandenseins oder der Abwesenheit eines Protein in einer Probe diagnostiziert werden kann.

Die Erfindung wird nachfolgend anhand von Ausführungsbeispielen und den dazugehörigen Figuren näher erläutert.

### Figuren

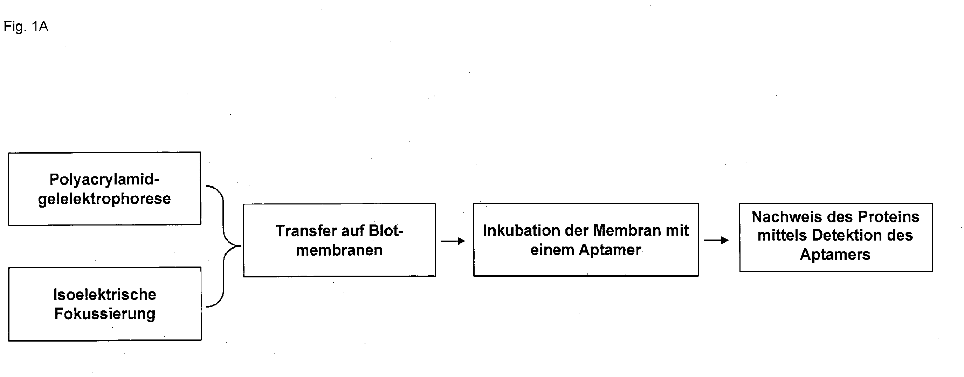

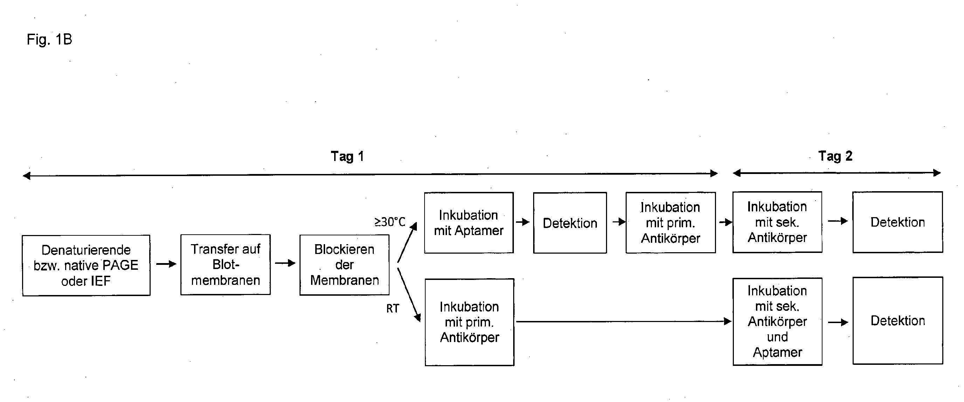

- Figur 1: Protokolle zum Nachweis von Proteinen im Aptamer Blot (A) oder im kombinierten Aptamer- und Western Blot (B).

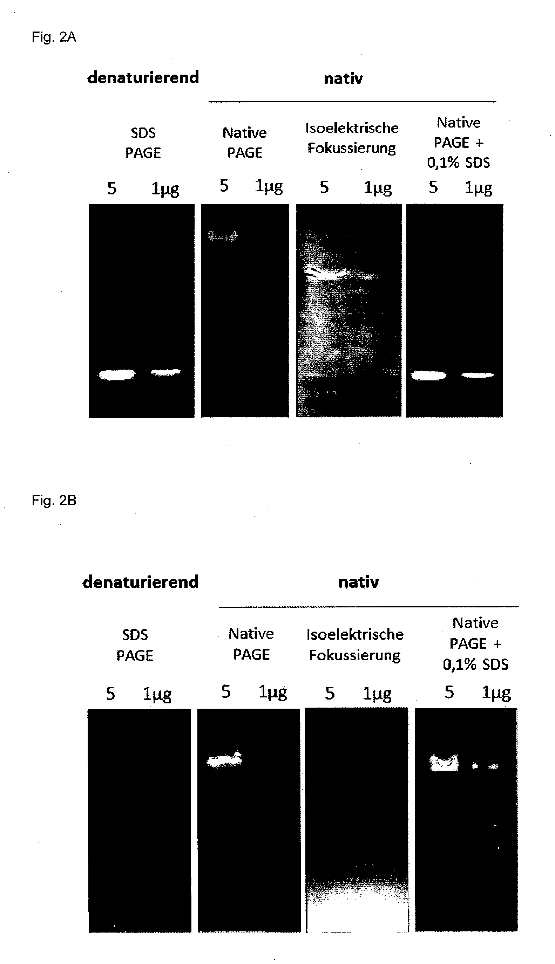

- Figur 2: Nachweis von α-Thrombin (A) und Streptavidin (B) nach Auftrennung unter nativen Bedingungen mittels spezifischer Aptamere. Zum Vergleich wurden α-Thrombin und Streptavidin auch entsprechend bekannter Protokolle unter denaturierenden Bedingungen aufgetrennt. Die Konzentration der FAM-markierten Aptamere betrug 1µg/ml.

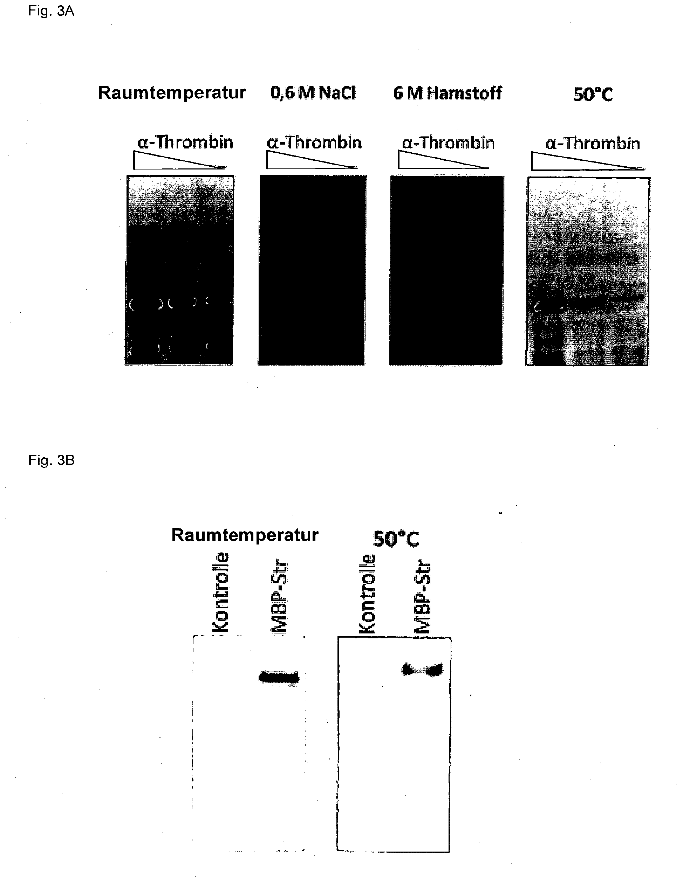

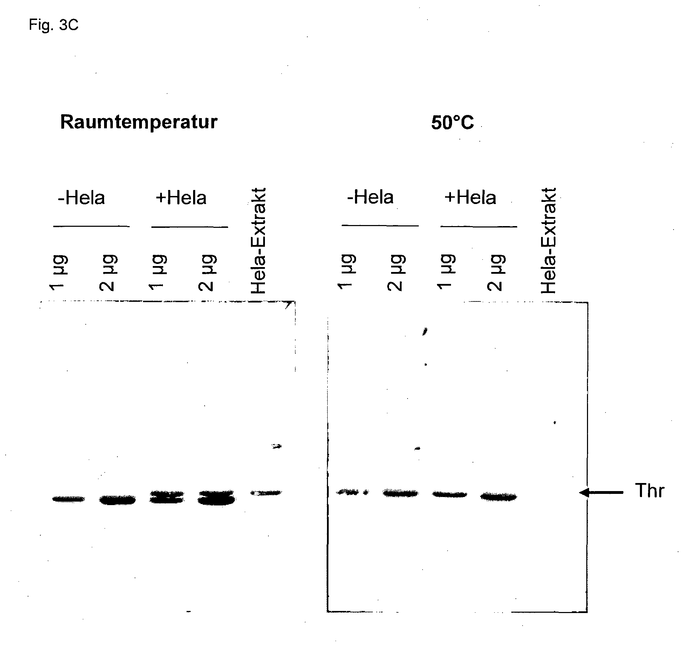

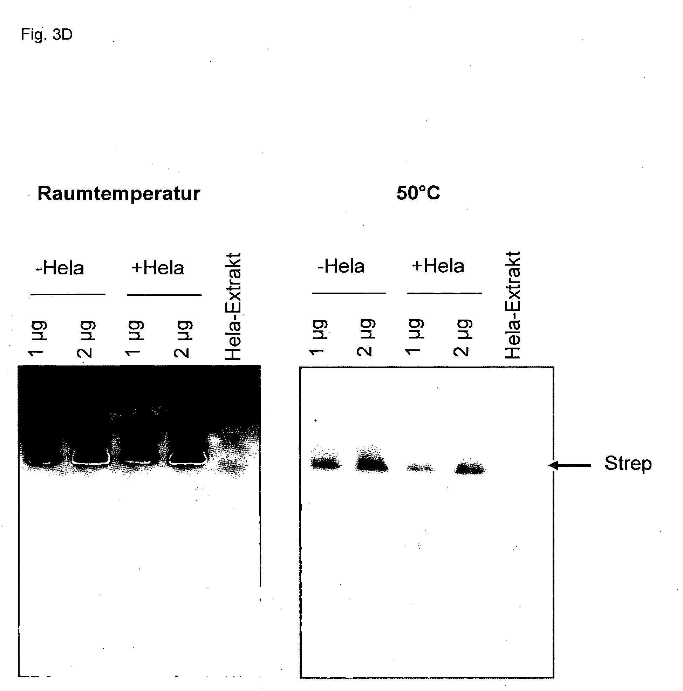

- Figur 3: Erhöhung der Spezifität des Aptamer Blots. Der Einfluss der Aptamerinkubation bei 50°C wurde mit einem Ansatz verglichen, der parallel bei Raumtemperatur inkubiert wurde. (A) 1µg und 2µg humanes α-Thrombin wurde mit HeLa-Extrakt gemischt und mittels Aptamer-Blot analysiert. Der Einfluss von 0,6M NaCl oder 6M Harnstoff wurde im Vergleich zu einer Inkubationstemperatur des Aptamers von 50°C und zum unbehandelten Ansatz bestimmt. (B) Streptavidin wurde als Fusionsprotein mit dem Maltosebindeprotein (MBP-Str) in *E.coli* exprimiert. Ein Zellextrakt des Expressionsstammes ohne MBP-Str-produzierendes Plasmid wurde als Kontrolle mitgeführt. (C) und (D) 1µg und 2µg Thrombin bzw. Streptavidin wurden mit HeLa Extrakt gemischt unter nativen Bedingungen inkl. 0,1% SDS aufgetrennt und mittels Aptamer Blot analysiert.

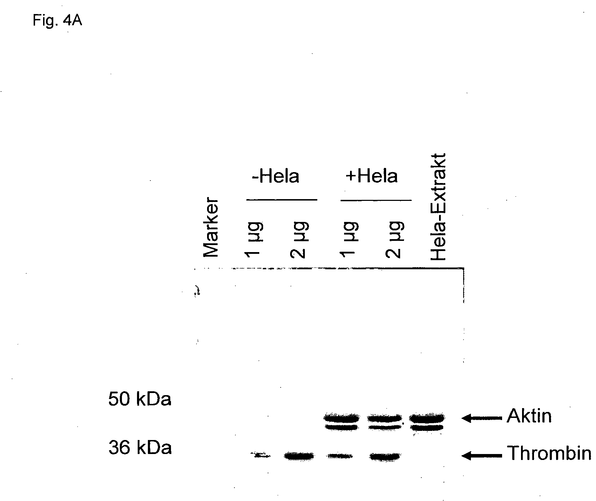

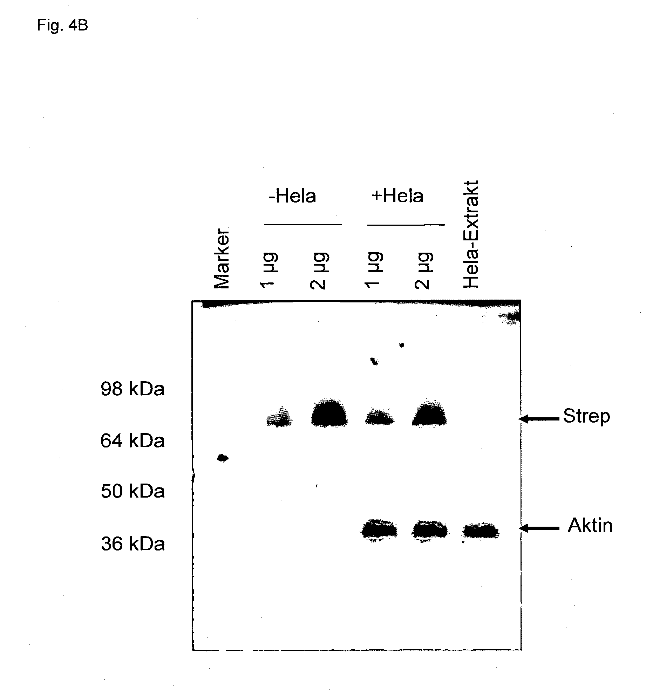

- Figur 4: Kombination von Western und Aptamer Blot. Humanes α-Thrombin (A) und Streptavidin (B) wurden mit HeLa-Extrakt gemischt und in einer nativen PAGE mit 0,1% SDS aufgetrennt. Nach dem Transfer der Proteine auf eine Membran und dem Blockieren der Membran erfolgte zunächst die Inkubation mit dem Erstantikörper gegen Aktin. Anschließend erfolgte die Behandlung der Membran mit dem Aptamer und dem Meerrettich-Peroxidase-markiertem Zweitantikörper bei Raumtemperatur.

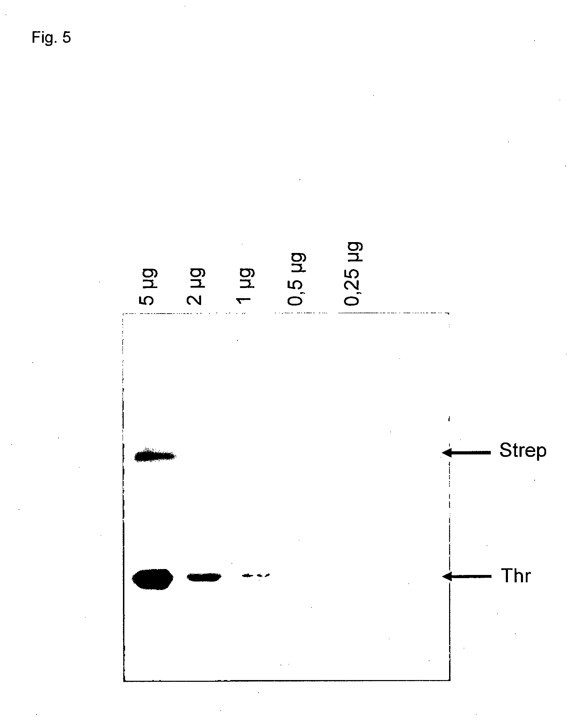

- Figur 5: Multiplex-Nachweis von Thrombin und Streptavidin im Aptamer-Blot. Thrombin wurde mit Streptavidin gemischt. Die Auftrennung des Proteingemisches erfolgte in einer nativen PAGE mit 0,1% SDS. Die Konzentration der Aptamere betrug jeweils 1µg/ml. Spur 1: je 5µg Thrombin/Streptavidin; Spur 2: je 2µg Thrombin/Streptavidin; Spur 3: je 1µg Thrombin/Streptavidin; Spur 4: je 0,5µg Thrombin/Streptavidin; Spur 5: je 0,25µg Thrombin/Streptavidin.

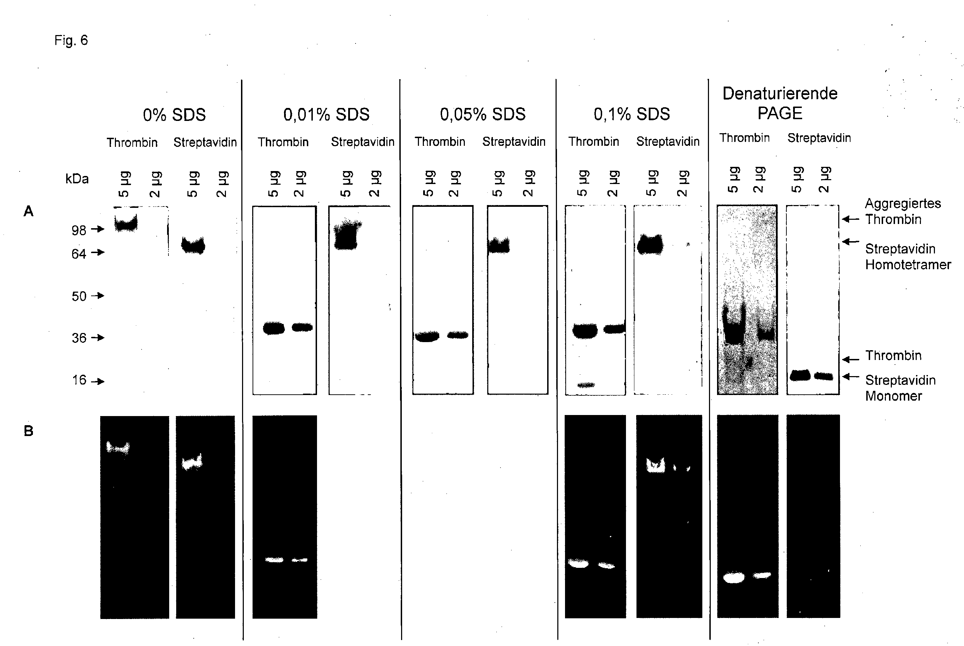

- Figur 6: Vergleich von nativer und denaturierender Gelelektrophorese von Thrombin und Streptavidin mit der nativen SDS-PAGE. Bei der nativen SDS-PAGE wurden verschiedene SDS-Mengen (0,01%; 0,05% 0,1%) im Lade- und Laufpuffer analysiert. (A) Coomassie-gefärbte Polyacrylamidgele; (B) Aptamer-Blot.

### Beispiele

Das erfindungsgemäße Verfahren dient dem Nachweis von Proteinen in proteinhaltigen Gemischen, wie beispielsweise Zellextrakten, Gewebeproben o.ä., mittels spezifischer Aptamere. Das Protokoll zum Nachweis der Proteine ist in Fig. 1A und 1B dargestellt.

Die für das Verfahren verwendeten Aptamere enthalten oder bestehen aus einer Nukleinsäuresequenz aus 15 Nukleotiden mit der SEQ ID No. 1 bzw. aus 40 Nukleotiden mit der SEQ ID No. 2. Das 15-mer: GGT TGG TGT GGT TGG (SEQ ID No. 1) und das 40-mer: ATC TCC GAT TGC CCC ACG ACG CAG TGG TCG GAG TTA CTT T (SEQ ID No. 2).

Die Auftrennung der Proteine kann mittels folgender Methoden erfolgen:
I. isoelektrischer Fokussierung
II. nativer Polyacrylamidgelelektrophorese (PAGE)
III. nativer PAGE inklusive 0,1% SDS.

Anschließend erfolgt der Transfer auf PVDF- oder Nitrocellulosemembranen. Die Membranen werden mit BSA blockiert und im Anschluss mit Fluoreszenz-markierten Aptameren inkubiert.

Um die Spezifität des Nachweises zu erhöhen, kann die Inkubation mit dem spezifischen Aptamer bei erhöhten Temperaturen (40°C, 45°C, 50°C) erfolgen. Die Detektion der Proteine erfolgt durch Fluoreszenzanregung bei der Fluorophor-spezifischen Wellenlänge.

Die gleichzeitige Detektion mehrerer Zielproteine (Multiplexanalyse) wird ermöglicht durch: I. Die Nutzung mehrerer Aptamere, die mit verschiedenen Fluorophoren konjugiert wurden. Die Inkubation der Blotmembranen mit den verschiedenen Aptameren erfolgt parallel. II. Die Kombination des Aptamer-Nachweises mit dem Antikörper-Nachweis. Beispielsweise kann die Inkubation des Aptamers bei Raumtemperatur erfolgen sowie parallel die Inkubation mit dem weiteren Antikörper. Erfolgt die Aptamer-Inkubation z.B. bei 40-60°C, kann die Inkubation mit dem Antikörper und dem weiteren Antikörper, nach der Detektion des Aptamers erfolgen.

Die drei genannten Punkte erlauben den schnellen und spezifischen Nachweis mehrerer Proteine in einem Ansatz. Damit hebt sich das etablierte Protokoll zum Aptamer Blot deutlich von den bislang beschriebenen Ansätzen ab.

### Detaillierte Protokolle:

Das Nachweisverfahren umfasst die folgenden Schritte: Schritt a) Auftrennung der Probe; Schritt b) Transfer der aufgetrennten Probe auf eine Membran; Schritt c) in Kontakt bringen der Membran mit einem Aptamer; Schritt d) Detektion des Aptamers.

Erfolgte die Auftrennung in Schritt a) mittels isoelektrischer Fokussierung (IEF) so wurde das entwickelte Protokoll mit den nachstehend aufgeführten Materialien und Bedingungen wie folgt durchgeführt:
Schritt a): Für die IEF wurde ein Gel mit einem pH-Gradienten von pH 3-10 verwendet (BioRad). Die Proben wurden mit Ladepuffer versetzt (Endkonzentrationen: 2 mM Lysin, 2 mM Arginin, 5% Glycerin) und auf das Gel aufgetragen. Anschließend wurde an das Gel eine elektrische Spannung angelegt und die entsprechende Laufzeit eingestellt (100V für 60 min; 250V für 60 min; 500V für 45 min). Der Kathodenpuffer enthielt 2mM Lysin und 2mM Arginin, der Anodenpuffer 0,7mM Phosphorsäure.
Schritt b): Als Blotmembran wurde eine PVDF-oder Nitrocellulosemembran benutzt. Der Transfer auf die Blotmembran erfolgte bei 10V für 60 min. Der verwendete Transferpuffer enthielt 0,7% Essigsäure.
   Anschließend wurde die Membran für 60 min bei Raumtemperatur geblockt mit 5% BSA im jeweiligen Selektionspuffer des Aptamers + 0,1% Tween.
Schritt c): Die Inkubation der Membran mit dem Aptamer erfolgte für 60 min bei Raumtemperatur bzw. bei 40°C, 45°C oder 50°C. Dafür wurde 0,5 - 1,5 µg/ml Aptamer im jeweiligen Selektionspuffer + 0,1% Tween + 1% BSA verwendet.
Schritt d): die Detektion erfolgte mittels Fluoreszenzanregung.

Erfolgte die Auftrennung in Schritt a) mittels Polyacrylamid-Gelelektrophorese (PAGE), so wurde das entwickelte Protokoll mit den nachstehend aufgeführten Materialien und Bedingungen wie folgt durchgeführt:
Schritt a): Die PAGE kann entweder unter nativen oder unter denaturierenden Bedingungen durchgeführt werden. Für die native PAGE (ohne SDS) wurde ein 10%iges Polyacrylamidgel verwendet. Die Proben wurden mit Ladepuffer versetzt (31,25 mM TrisCl; pH 6,8 + 5% Glycerin + Bromphenolblau) und auf das Gel aufgetragen. Anschließend wurde an das Gel eine elektrische Spannung angelegt und die entsprechende Laufzeit eingestellt (100V für 60 min). Als Laufpuffer wurde ein Tris/Glycin-Puffer verwendet.
   Der nativen PAGE kann eine geringe Menge an SDS beigefügt werden. Hierfür wurde ein 10%iges Polyacrylamidgel verwendet. Auf dieses Gel wurde die 0,1 % SDS-enthaltene Probe (Probe + 31,25 mM TrisCI pH 6,8 + 5% glycerol + 0,1 % SDS + Bromphenolblau) aufgetragen. Anschließend wurde an das Gel eine elektrische Spannung angelegt und die entsprechende Laufzeit eingestellt (100V für 60 min). Als Laufpuffer wurde ein Tris/Glycin-Puffer mit 0,1% SDS verwendet.
   Für die denaturierende PAGE wurde ein 10%iges BisTris-Polyacrylamidgel mit verwendet. Auf dieses Gel wurde die denaturierte Probe (Probenpuffer: 315 mM Tris pH 6,8, 10 % SDS, 50 % Glycerin, 0,05% Bromphenolblau, 25 % β-Mercaptoethanol) aufgetragen. Anschließend wurde an das Gel eine elektrische Spannung angelegt und die entsprechende Laufzeit eingestellt (200V für 30 min). Als Laufpuffer wurde ein MOPS/Tris-Puffer mit 5 mM EDTA, 1 mM Natriumbisulfit und 0,1% SDS verwendet.
Schritt b): Als Blotmembran wurde eine PVDF- oder Nitrocellulosemembran benutzt. Der Transfer auf die Blotmembran erfolgte bei 140 mA für 70 min. Der verwendete Transferpuffer enthielt 25 mM Tris, 192 mM Glycin und 20% Methanol.
   Anschließend wurde die Membran für 60 min bei Raumtemperatur geblockt mit 5% BSA im jeweiligen Selektionspuffer des Aptamers + 0,1% Tween20.
Schritt c): Die Inkubation der Membran mit dem Aptamer erfolgte für 60 min bei Raumtemperatur bzw. bei 40°C, 45°C oder 50°C. Dafür wurde 0,5 - 1,5 µg/ml Aptamer im jeweiligen Selektionspuffer + 0,1% Tween20 + 1% BSA verwendet.
Schritt d): die Detektion erfolgte mittels Fluoreszenzanregung oder ECL-Entwicklung.

Auch die Kombination des Aptamer Blots mit dem klassischen Western Blot ist mit Hilfe des entwickelten Protokolls möglich. Die Inkubation mit dem spezifischen Aptamer erfolgt vor der Behandlung der Proteine mit Erst- und Zweitantikörper, sofern das Aptamer bei höheren Temperaturen inkubiert wurde (Fig. 1B). Wurde die Membran bei Raumtemperatur mit der Aptamer-Lösung inkubiert, dann wurde die Membran zunächst mit dem Erstantikörper inkubiert und anschließend parallel mit dem Aptamer und dem Zweitantikörper. Anschließend erfolgt die parallele Detektion von Aptamer und Antikörper.

Das Nachweisverfahren umfasst die folgenden Schritte, wenn die Membran zuerst mit Aptamer und anschließend mit den Antikörpern inkubiert wurde: Schritt a) Auftrennung der Probe; Schritt b) Transfer der aufgetrennten Probe auf eine Membran; Schritt c) in Kontakt bringen der Membran mit einem Aptamer; Schritt d) Detektion des Aptamers, Schritt i) in Kontakt bringen der Membran mit dem primären Antikörper, Schritt ii) in Kontakt bringen der Membran mit dem sekundären Antikörper, sowie Detektion des Antikörpers.

Das entwickelte Protokoll mit den nachstehend aufgeführten Materialien und Bedingungen wie folgt durchgeführt:
Schritt a): Es wurde ein 10%iges Polyacrylamidgel verwendet. Auf dieses Gel wurde die 0,1 % SDS-enthaltene Probe aufgetragen (Ladepuffer: 31,25 mM TrisCI pH 6,8 + 5% Glycerin + 0,1 % SDS + Bromphenolblau). Anschließend wurde an das Gel eine elektrische Spannung angelegt und die entsprechende Laufzeit eingestellt (100V für 60 min). Als Laufpuffer wurde ein Tris/Glycin-Puffer mit 0,1% SDS verwendet.
Schritt b): Als Blotmembran wurde eine PVDF-oder Nitrocellulosemembran benutzt. Der Transfer auf die Blotmembran erfolgte bei 140 mA für 70 min. Der verwendete Transferpuffer enthielt 25 mM Tris, 192 mM Glycin und 20% Methanol.
   Anschließend wurde die Membran für 60 min bei Raumtemperatur geblockt mit 5% BSA im jeweiligen Selektionspuffer des Aptamers + 0,1% Tween20.
Schritt c): Die Inkubation der Membran mit dem Aptamer erfolgte für 60 min bei Temperaturen ≥40°C. Dafür wurde 0,5 - 1,5µg/ml Aptamer im jeweiligen Selektionspuffer + 0,1% Tween20 + 1% BSA verwendet.
Schritt d): Die Detektion erfolgte mittels Fluoreszenzanregung.
   Schritt i): Inkubation mit primärem Antikörper bei Raumtemperatur für 1 h oder ggfs bei 4°C über Nacht.
   Schritt ii): Inkubation mit sekundärem Antikörper, bei Raumtemperatur für 1 h. Die Detektion erfolgte mittels ECL-Methode.

Das Nachweisverfahren umfasst die folgenden Schritte, wenn die Membran zuerst mit Erstantikörper und anschließend mit parallel mit dem Sekundärantikörper und dem Aptamer inkubiert wurde: Schritt a) Auftrennung der Probe; Schritt b) Transfer der aufgetrennten Probe auf eine Membran; Schritt i) in Kontakt bringen der Membran mit einem Erstantikörper; Schritt c) in Kontakt bringen der Membran mit dem Aptamer und dem sekundären Antikörper, Schritt d) bzw. ii) Detektion des Antikörpers und des Aptamers

Das entwickelte Protokoll mit den nachstehend aufgeführten Materialien und Bedingungen wie folgt durchgeführt:
Schritt a): Es wurde ein 10%iges Polyacrylamidgel verwendet. Auf dieses Gel wurde die 0,1 % SDS-enthaltene Probe aufgetragen (Ladepuffer: 31,25 mM TrisCI pH 6,8 + 5% glycerol + 0,1 % SDS + Bromphenolblau). Anschließend wurde an das Gel eine elektrische Spannung angelegt und die entsprechende Laufzeit eingestellt (100V für 60 min). Als Laufpuffer wurde ein Tris/Glycin-Puffer mit 0,1% SDS verwendet.
Schritt b): Als Blotmembran wurde eine PVDF-oder Nitrocellulosemembran benutzt. Der Transfer auf die Blotmembran erfolgte bei 140 mA für 70 min. Der verwendete Transferpuffer enthielt 25 mM Tris, 192 mM Glycin und 20% Methanol.
   Anschließend wurde die Membran für 60 min bei Raumtemperatur geblockt mit 5% BSA im jeweiligen Selektionspuffer des Aptamers + 0,1% Tween20.
   Schritt i): Die Inkubation der Membran mit dem primären Antikörper erfolgte bei Raumtemperatur für 1 h oder ggfs bei 4°C über Nacht.
Schritt c): Die Inkubation der Membran mit dem Aptamer und dem sekundären Antikörper erfolgte für 60 min bei Raumtemperatur. Dafür wurde 0,5 - 1,5µg/ml Aptamer und ein sekundärer Antikörper im Selektionspuffer des Aptamers + 0,1% Tween20 + 1% BSA verwendet.
Schritt d) bzw. ii): Die Detektion erfolgte mittels Fluoreszenzanregung und ECL-Entwicklung.

### Beispiel 1: Vergleich von denaturierender und nativer PAGE und isoelektrischer Fokussierung als Trennmethode für den Aptamer Blot (Fig. 2)

Die Interaktion von Aptamer und Protein ist sowohl sequenz- als auch strukturspezifisch. Entsprechend sollten native Bedingungen für die Trennung von Proteinen für Aptamer-BlotAnwendungen von Vorteil sein. Entsprechend wurde der Aptamer-basierte Nachweis von Thrombin und Streptavidin nach Trennung unter nativen bzw. denaturierenden Bedingungen verglichen (Fig. 2).

Bislang findet sich in der Literatur für Thrombin nur die Auftrennung in der denaturierenden PAGE. Da der Aptamer Blot unter diesen denaturierenden Bedingungen funktioniert, kann - davon ausgegangen werden, dass die Wechselwirkung von Thrombin und dem Thrombinbindenden Aptamer (TBA) ausschließlich auf sequenz-spezifischen Wechselwirkungen beruht (Fig. 2A). Der Nachweis von Streptavidin in der denaturierenden PAGE war allerdings nicht möglich (Fig. 2B). In Coomassie-gefärbten Gelen nach denatuierender PAGE war zu erkennen, dass Streptavidin in momomerer Form vorlag und somit die homotetramere Form des nativen Proteins zerstört wurde. Da Streptavidin in denaturierter Form nicht mehr nachweisbar war, kann davon ausgegangen werden, dass die Wechselwirkung zwischen Streptavidin und dessen spezifischen Aptamer auf strukturellen Wechselwirkungen beruht.

Thrombin wurde ebenfalls nach nativer PAGE im Aptamer-Blot analysiert (Fig. 2A). Im Vergleich zur denaturierenden PAGE wurden nur sehr schwache Signale detektiert, die zudem eine Größe weit über 100 kDa aufwiesen. Dies lässt sich vermutlich auf die Ausbildung von Aggregaten zurückführen. Die Ausbildung der Aggregate führte zu einem erschwerten Einlaufen in das PAGE-Gel und zum anderen zu einem sehr geringen Transfer auf die PVDF-Membran. Auch Streptavidin wurde in einer nativen PAGE aufgetrennt und anschließend im Aptamer-Blot analysiert (Fig. 2B). Nach dem Transfer des Proteins auf PVDF-Membranen war die Detektion mittels Streptavidin-bindenden Aptamers (SBA) möglich.

Weiterhin wurde die isoelektrische Fokussierung (IEF) auf ihre Anwendbarkeit für den Aptamer-Blot getestet. Sowohl Thrombin als auch Streptavidin wurden auf IEF Gele aufgetragen. Die Fokussierung wurde zunächst mittels Coomassie-Färbung analysiert (Daten nicht gezeigt). Sowohl Thrombin als auch Streptavidin sind in die IEF-Gele eingelaufen. Während Thrombin im Aptamer-Blot sehr gut nachweisbar war (Fig. 2A), konnten mit dem SBA keine Banden detektiert werden (Fig. 2B).

Es wurde eine weitere native Trennmethode analysiert, bei der die Proben vor der PAGE weder durch Hitze noch durch reduzierende Agenzien denaturiert wurden (Fig. 2A und 2B, rechts). Der Lade- und Laufpuffer wurde allerdings mit 0,1% SDS versetzt, sodass eine gleichmäßige negative Beladung der Proteine erzielt und die Ausbildung von Aggregaten verhindert wurde. Eine Denaturierung der Proteine wird unter diesen Bedingungen allerdings nicht beobachtet. Nach der Analyse von Thrombin und Streptavidin im Aptamer Blot unter diesen Bedingungen wurden deutliche Signale beobachtet. Die Streptavidin-Homotetramere wurden durch diese Behandlung nicht zerstört. Diese Methode scheint entsprechend am Besten geeignet, um Proteine mittels Aptamer nachzuweisen.

### Beispiel 2: Nachweis von humanem α-Thrombin und Streptavidin in Proteingemischen (Fig. 3)

Humanes α-Thrombin wurde einem HeLa-Zellextrakt zugegeben, während Streptavidin als Fusionsprotein mit dem Maltosebindeprotein (MBP) in *E.coli* exprimiert wurde. Das α-Thrombin/HeLa-Gemisch wurde in einer denaturierenden SDS-PAGE aufgetrennt, das Streptavidin/*E*.*coli*-Gemisch wurde in einer nativen PAGE aufgetrennt.

Beim Nachweis von α-Thrombin zeigte es sich, dass neben α-Thrombin auch eine Reihe von HeLa-Proteinen detektiert wurden (Fig. 3A, links). Um die Zahl der unspezifischen Bindungen zu reduzieren, wurden im Anschluss an die Aptamerinkubation verschiedene Waschschritte durchgeführt. Zum einen wurden die Membranen mit 0,6M NaCl gewaschen und zum Anderen mit 6M Harnstoff. Die unspezifischen Bindungen wurden durch die jeweiligen Waschschritte reduziert, allerdings nahm im gleichen Maße auch das spezifische α-Thrombin-Signal ab und der Hintergrund wurde dunkler (Fig. 3A, Mitte). Entsprechend waren diese beiden Waschschritte für eine Erhöhung der Spezifität im Aptamer Blot nicht geeignet.

Erfolgte die Aptamer-Inkubation jedoch bei einer Temperatur von 50°C, zeigte sich ein stark reduzierter Hintergrund bei nur leicht verringerter Stärke des α-Thrombin-Signals (Fig. 3A, rechts).

Nach heterologer Expression und nativer PAGE von Streptavidin in *E.coli* wurden deutlich weniger unspezifische Banden als für α-Thrombin im HeLa-Gemisch beobachtet (Fig. 3B, links). Um die Spezifität des Nachweises zu erhöhen, wurde auch hier die Membran bei 50°C mit dem Streptavidin-Aptamer inkubiert. Eine Reduktion unspezifischer Banden war detektierbar (Fig. 3B, rechts).

Da sich gezeigt hatte, dass eine Trennung von humanem α-Thrombin und Streptavidin in nativen Acrylamidgelen und der Nutzung von 0,1% SDS zu sehr guten Ergebnissen im Aptamer-Blot führten, wurde auch analysiert, ob eine Inkubation bei erhöhten Temperaturen zu spezifischeren Ergebnissen führt (Abb. 3C und 3D). Entsprechend wurden Thrombin oder Streptavidin mit Hela-Extrakt gemischt und mittels nativer PAGE + 0,1% SDS aufgetrennt. Anschließend wurden die Proteine auf PVDF-Membranen übertragen, die Membranen wurden mit BSA blockiert und anschließend mit TBA oder SBA inkubiert. Während mit Thrombin unspezifische Signale bei der Aptamer-Inkubation bei Raumtemperatur beobachtet wurden, erlaubte die Inkubation bei 50°C eine deutlich spezifischere Detektion von Thrombin (Abb. 3C). Mit dem Streptavidin-bindenden Aptamer gab es weder unspezifische Signale bei der Inkubation bei Raumtemperatur noch bei 50°C (Abb. 3D). Das SBA scheint deutlich spezifischer zu sein als das TBA.

### Beispiel 3: Kombination von Aptamer- und Western Blot (Fig. 4)

Da bislang deutlich mehr Antikörper als Aptamere zum Nachweis von Proteinen zur Verfügung stehen, wurde die Kompatibilität von Western und Aptamer Blot analysiert. Hierzu wurden sowohl humanes α-Thrombin als auch Streptavidin mit Hela-Extrakt gemischt und anschließend wurde Aktin mittels Western Blot und Thrombin mit Hilfe des Aptamer Blots untersucht.

Die Proteingemische wurden in einer nativen PAGE mit 0,1% aufgetrennt. Anschließend erfolgte der Transfer der Proteine auf eine PVDF-Blotmembran und die Blockierung der Membran mit BSA. Danach wurde die Membran mit dem Erstantikörper gegen Aktin über Nacht inkubiert. Im Anschluss erfolgte die Inkubation der Membran bei Raumtemperatur mit dem TBA oder SBA und einem Zweitantikörper, der mit der Meerrettich-Peroxidase konjugiert war. Abschließend erfolgte die parallele Detektion von Aptamer und Sekundärantikörper.

Mit diesem Ansatz war es möglich, sowohl Thrombin als auch Streptavidin gemeinsam mit Aktin nachzuweisen (Fig. 4). In den Ansätzen ohne Thrombin oder Streptavidin wurden keine Signale im entsprechenden Molekulargewichtsbereich detektiert. Die erfolgreiche Kombination von Western und Aptamer Blot konnte gezeigt werden.

### Beispiel 4: Multiplex-Nachweis von Thrombin und Streptavidin (Fig. 5)

Durch die Nutzung fluoreszenz-markierter Aptamere wird der parallele Nachweis mehrerer Proteine in einem Ansatz möglich. Die Machbarkeit wurde exemplarisch für ein Thrombin-Streptavidin Gemisch gezeigt (Fig. 5). Das TBA wurde mit Cy5 markiert und das SBA mit FAM. Die Auftrennung des Proteingemisches erfolgte in der nativen PAGE inklusive 0,1% SDS. Nach dem Transfer der Proteine auf PVDF-Membranen und der gemeinsamen Inkubation mit beiden Aptameren wurden deutliche Signale sowohl für Thrombin als auch für Streptavidin beobachtet. Die Nachweisgrenze für Streptavidin lag bei 0,25 µg, die für Thrombin bei 0,5 µg.

### Beispiel 5: Vergleich von nativen und denaturierenden Trennverfahren mit der nativen SDS-PAGE (Fig. 6)

Vergleicht man das Laufverhalten von Thrombin in Polyacrylamidgelen nach Coomassie-Färbung stellt man fest, dass Thrombin unter nativen Bedingungen Aggregate bildet (Fig. 6A). Die Ausbildung von Aggregaten führt im Aptamer-Blot zu sehr schwachen Signalen (Fig. 6B). Bereits die Zugabe geringer SDS Mengen (ab 0,01% SDS) zum Lade- und Laufpuffer während der Gelelektrophorese verhindern die Ausbildung der Aggregate. Der Thrombin-Nachweis wird so deutlich sensitiver und so kann davon ausgegangen werden, dass die native Struktur des Thrombins erhalten bleibt.

Strepatvidin liegt als natives, funktionelles Protein als Homotetramer vor. Diese homotetramere Struktur lässt sich auch sehr gut in Coomassie-gefärbten Polyacrylamidgelen nachweisen. Unter nativen Bedingungen und nach der Zugabe von 0,01%, 0,05% oder 0,1% SDS zum Lade- und Laufpuffer war zu erkennen, dass Streptavidin als natives Protein in die Gele eingelaufen ist, da das Protein bezogen auf einen Marker bei einer Größe von ca. 64 kDa detektiert wurde (Fig. 6A). Unter denaturierenden Bedingungen waren nur die Streptavidin-Monomere im Gel zu sehen. Das führte dazu, dass der Streptavidin-Nachweis unter denaturierenden Bedingungen im Aptamer-Blot nicht möglich war (Fig. 6B). In der nativen PAGE bzw. der nativen SDS-PAGE mit 0,1% war der Nachweis von Streptavidin im Aptamer-Blot hingegen möglich.

## Patentansprüche

1. Verfahren zum in vitro Nachweis eines ersten Proteins in einer Probe, umfassend die Schritte:
a) Auftrennen der Probe mittels eines nativen Trennverfahrens;
b) Transfer der aufgetrennten Probe auf eine Membran;
c) In Kontakt bringen der Membran mit einem Aptamer, das spezifisch an das erste Protein bindet und
d) Nachweis des ersten Proteins mittels Detektion des Aptamers, das an das erste Protein gebunden vorliegt
**dadurch gekennzeichnet, dass** das in Kontakt bringen der Membran mit dem Aptamer in Schritt c) bei einer Temperatur von ≥ 30°C erfolgt.

2. Verfahren nach Anspruch 1, wobei das native Trennverfahren eine elektrophoretische Auftrennung ist, bevorzugt eine Gelelektrophorese.

3. Verfahren nach Anspruch 1 oder 2, wobei das native Trennverfahren dadurch charakterisiert ist, dass eine SDS-Konzentration von 0% bis 0,2 % SDS, bevorzugt 0,01% bis 0,15% SDS, besonders bevorzugt 0,1% SDS verwendet wird.

4. Verfahren nach einem der Ansprüche 1 bis 3, wobei das in Kontakt bringen der Membran mit dem Aptamer in Schritt c) bei einer Temperatur von 30°C bis 70°C, besonders bevorzugt bei 40°C bis 60°C, ganz besonders bevorzugt bei 40°C bis 50°C.

5. Verfahren nach einem der Ansprüche 1 bis 4, wobei die Probe ein proteinhaltiges Gemisch umfasst.

6. Verfahren nach Anspruch 5, wobei das proteinhaltige Gemisch aus einer Zellkultursupension, einem Zellkulturüberstand, einer Gewebeprobe oder einer Körperflüssigkeit erhalten wird oder eine Zellkultursuspension, einen Zellkulturüberstand, eine Gewebeprobe oder eine Körperflüssigkeit umfasst.

7. Verfahren nach einem der Ansprüche 1 bis 6, wobei das Aptamer eine Modifikation aufweist ausgewählt aus einer Biotin-Markierung, Fluoreszenzmarkierung, Radioisotop-Markierung, Digoxigenin-Markierung, Peroxidase-Markierung, alkalischer Phosphatase-Markierung oder einer Markierung mit Nanopartikeln.

8. Verfahren nach einem der Ansprüche 1 bis 7, wobei die Detektion des Aptamers direkt oder indirekt erfolgen kann.

9. Verfahren nach einem der Ansprüche 1 bis 8 zum zusätzlichen Nachweis eines zweiten Proteins in der Probe, wobei zusätzlich:
i. nach Schritt b) die Membran mit einem Bindungsmolekül, das spezifisch an das zweite Protein bindet, in Kontakt gebracht wird; und
ii. der Nachweis des zweiten Proteins mittels Detektion des Bindungsmoleküls, das an das zweite Protein gebunden vorliegt, erfolgt.

10. Verfahren nach Anspruch 9, wobei das Bindungsmolekül eine Modifikation aufweist ausgewählt aus einer Biotin-Markierung, Fluoreszenzmarkierung, Radioisotop-Markierung, Digoxigenin-Markierung, Peroxidase-Markierung, alkalischer Phosphatase-Markierung oder einer Markierung mit Nanopartikeln.

11. Verfahren nach Anspruch 9 oder 10, wobei das Bindungsmolekül ein weiteres Aptamer oder ein Antikörper ist.

## Claims

1. A method for the in-vitro detection of a first protein in a sample, comprising the steps of:
a) separating the sample by means of a native separation process;
b) transferring the separated sample onto a membrane;
c) contacting the membrane with an aptamer that specifically binds to the first protein; and
d) detecting the first protein by means of detecting the aptamer bound to the first protein,
**characterised in that** the contacting of the membrane with the aptamer in step c) is performed at a temperature of ≥ 30 °C.

2. The method of claim 1, wherein the native separation process is an electrophoretic separation, preferably a gel electrophoresis.

3. The method of claim 1 or 2, wherein the native separation process is **characterised in that** an SDS concentration of 0 % to 0.2 % SDS, preferably of 0.01 % to 0.15 % SDS, particularly preferably of 0.1 % SDS, is used.

4. The method of any one of claims 1 to 3, wherein the contacting of the membrane with the aptamer in step c) is performed at a temperature of 30 °C to 70 °C, particularly preferably of 40 °C to 60 °C, most preferably of 40 °C to 50 °C.

5. The method of any one of claims 1 to 4, wherein the sample comprises a protein-containing mixture.

6. The method of claim 5, wherein the protein-containing mixture is obtained from a cell culture suspension, a cell culture supernatant, a tissue sample or a humour, or comprises a cell culture suspension, a cell culture supernatant, a tissue sample or a humour.

7. The method of any one of claims 1 to 6, wherein the aptamer has a modification selected from a biotin marker, a fluorescence marker, a radioisotope marker, a digoxigenin marker, a peroxidase marker, an alkaline phosphatase marker, or a marker with nanoparticles.

8. The method of any one of claims 1 to 7, wherein the detection of the aptamer may be performed directly or indirectly.

9. The method of any one of claims 1 to 8 for the additional detection of a second protein in the sample, wherein additionally:
i. after step b), the membrane is contacted with a bonding molecule that specifically binds to the second protein; and
ii. the detection of the second protein is performed by means of detecting the bonding molecule bound to the second protein.

10. The method of claim 9, wherein the bonding molecule has a modification selected from a biotin marker, a fluorescence marker, a radioisotope marker, a digoxigenin marker, a peroxidase marker, an alkaline phosphatase marker, or a marker with nanoparticles.

11. The method of claim 9 or 10, wherein the bonding molecule is a further aptamer or an antibody.

## Revendications

1. Procédé pour la détection in vitro d'une première protéine dans un échantillon, comprenant les étapes :
a) Séparation de l'échantillon au moyen d'un procédé de séparation natif ;
b) Transfert de l'échantillon séparé sur une membrane ;
c) Mise en contact de la membrane avec un aptamère, lequel se lie de façon spécifique à la première protéine, et
d) Détection de la première protéine au moyen d'une détection de l'aptamère qui est lié à la première protéine
**caractérisé en ce que** la mise en contact de la membrane et de l'aptamère à l'étape c) est effectuée à une température ≥ 30°C.

2. Procédé selon la revendication 1, le procédé de séparation natif étant une séparation électrophorétique, de préférence une électrophorèse sur gel.

3. Procédé selon la revendication 1 ou 2, le procédé de séparation natif étant **caractérisé en ce qu'**une concentration SLS de 0% à 0,2 % SLS, de préférence de 0,01% à 0,15% SLS, en particulier de préférence de 0,1% SLS est utilisée.

4. Procédé selon l'une des revendications 1 à 3, la mise en contact de la membrane et de l'aptamère lors de l'étape c) étant effectuée à une température de 30°C à 70°C, en particulier de préférence de 40°C à 60°C, tout particulièrement de préférence de 40°C à 50°C.

5. Procédé selon l'une des revendications 1 à 4, l'échantillon comprenant un mélange protéique.

6. Procédé selon la revendication 5, le mélange protéique étant obtenu à partir d'une suspension de culture cellulaire, un surnageant de culture cellulaire, un échantillon de tissu ou un fluide corporel ou comprend une suspension de culture cellulaire, un surnageant de culture cellulaire, un échantillon de tissu ou un fluide corporel.

7. Procédé selon l'une des revendications 1 à 6, l'aptamère présentant une modification sélectionnée parmi le groupe se composant de : marquage par biotine, marquage par fluorescence, marquage par radioisotope, marquage par digoxigénine, marquage par peroxydase, marquage par phosphatase alcaline ou un marquage par nanoparticules.

8. Procédé selon l'une des revendications 1 à 7, la détection de l'aptamère pouvant être effectuée directement ou indirectement.

9. Procédé selon l'une des revendications 1 à 8, pour la détection supplémentaire d'une deuxième protéine dans l'échantillon, de plus :
i. Après l'étape b), la membrane est mise en contact avec une molécule de liaison, laquelle se lie spécifiquement à la deuxième protéine ; et
ii. La détection de la deuxième protéine est effectuée au moyen d'une détection de la molécule de liaison qui est liée à la deuxième protéine.

10. Procédé selon la revendication 9, la molécule de liaison présentant une modification sélectionnée parmi le groupe se composant de : marquage par biotine, marquage par fluorescence, marquage par radioisotope, marquage par digoxigénine, marquage par peroxydase, marquage par phosphatase alcaline ou un marquage par nanoparticules.

11. Procédé selon la revendication 9 ou 10, la molécule de liaison étant un autre aptamère ou un anticorps.
